# EUROPEAN PATENT SPECIFICATION

(11) **EP 1 990 052 B1**
(45) Date of publication and mention of the grant of the patent: **16.05.2012**
(21) Application number: 07737396.7
(22) Date of filing: 26.02.2007
(51) Int. Cl.: A61K 31/4245, A61J 1/03, A61P 9/12

(54) **PHARMACEUTICAL PACKAGE COMPRISING 2-ETHOXY-1-{[2'-(5-OXO-4,5-DIHYDRO-1,2,4-OXADIAZOL-3-YL)BIPHENYL-4-YL]METHYL}-1H-BENZIMIDAZOLE-7-CARBOXYLIC ACID (5-METHYL-2-OXO-1,3-DIOXOL-4-YL)METHYL OR 2-CYCLOPROPYL-1-{[2'-(5-OXO-4,5-DIHYDRO-1,2,4-OXADIAZOL-3-YL)BIPHENYL-4-YL]METHYL}-1H-BENZIMIDAZOLE-7-CARBOXYLIC ACID (5-METHYL-2-OXO-1,3-DIOXOL-4-YL)METHYL AND A DESICCANT.**
PHARMAZEUTISCHE PACKUNG ENTHALTEND (5-METHYL-2-OXO-1,3-DIOXOL-4-YL)METHYL-2-ETHOXY-1-{[2'-(5-OXO-4,5-DIHYDRO-1,2,4-OXADIAZOL-3-YL)BIPHENYL-4-YL]METHYL}-1H-BENZIMIDAZOL-7-CARBOXYLAT ODER (5-METHYL-2-OXO-1,3-DIOXOL-4-YL)METHYL-2-CYCLOPROPYL-1-{[2'-(5-OXO-4,5-DIHYDRO-1,2,4-OXADIAZOL-3-YL)BIPHENYL-4-YL]METHYL}-1H-BENZIMIDAZOL-7-CARBOXYLAT UND EIN TROCKENMITTEL
EMBALLAGE PHARMACEUTIQUE COMPRENANT DU 2-ETHOXY-1-{[2'-(5-OXO-4,5-DIHYDRO-1,2,4-OXADIAZOL-3-YL)BIPHENYL-4-YL]METHYL}-1H-BENZIMIDAZOLE-7-CARBOXYLATE DE (5-METHYL-2-OXO-1,3-DIOXOL-4-YL)METHYLE OU DU 2-CYCLOPROPYL-1-{[2'-(5-OXO-4,5-DIHYDRO-1,2,4-OXADIAZOL-3-YL)BIPHENYL-4-YL]METHYL}-1H-BENZIMIDAZOLE-7-CARBOXYLATE DE (5-METHYL-2-OXO-1,3-DIOXOL-4-YL)METHYLE ET UN AGENT DESHYDRATANT

(30) Priority: 27.02.2006 US 776686 P
(43) Date of publication of application: 12.11.2008
(73) Proprietor: Takeda Pharmaceutical Company Limited, Osaka-shi, Osaka 541-0045 (JP)
(72) Inventor: NONOMURA, Koji, Osaka-shi, Osaka 532-8686 (JP)
(74) Representative: Dossmann, Gérard
(86) International application number: PCT/JP2007/053544
(87) International publication number: WO 2007/097451

(56) References cited:
- WO-A-2008/041663
- WO-A1-2006/115264
- WO-A2-2006/107062
- JP-A- 02 243 156
- JP-A- 04 071 560
- JP-A- 2005 272 451
- US-A1- 2005 187 269

## Description

### Technical Field

The present invention relates to a pharmaceutical package with decreased uncomfortable odor.

### Background of the Invention

2-Ethoxy-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl, which is a prodrug of a benzimidazole derivative having a strong angiotensin II receptor antagonistic action, 2-ethoxy-1-{[2'-(5-oxo-4,5-dihydro-1,2,4-oxadiazol-3-yl)biphenyl-4-yl]methyl}-1H-benzimidazole-7-carboxylic acid, and a salt thereof (hereinafter to be sometimes referred to as "compound A"; patent reference 1: WO2005/080384), and 2-cyclopropyl-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl, which is a prodrug of 2-cyclopropyl-1-{[2'-(5-oxo-4,5-dihydro-1,2,4-oxadiazol-3-yl)biphenyl-4-yl]methyl}-1H-benzimidazole-7-carboxylic acid, and a salt thereof (hereinafter to be sometimes referred to as "compound B"; patent reference 2: WO2006/107062) are considered to be promising as therapeutic drugs for hypertension and the like.

While olmesartan medoxomil (patent reference 3: JP-A-5-78328), which is a monocyclic imidazole derivative, has already been used clinically as a therapeutic drug for hypertension, olmesartan medoxomil is known to emit a peculiar odor.

Needless to say, effectiveness and safety are most important for pharmaceutical products; however, convenience is also important from practical aspects. For example, taking oral tablet, which is most popular as a form of a pharmaceutical product, as an example, the size, taste, smell (odor), appearance, texture and the like of the tablet are also important for patients taking the tablet each day.

As a method of decreasing an uncomfortable odor, a decomposition method, an adsorption method, a masking method and the like are known. In the decomposition method, a substance responsible for the odor is decomposed, and the method includes decomposition by ozone, decomposition by catalyst, decomposition by pharmaceutical agent and the like. In the adsorption method, a substance responsible for the odor is adsorbed, and the method includes adsorption by activated carbon, a method including adsorption to an electric field applied with a high voltage and the like. In the masking method, aromatic and the like are used to prevent direct smell of an uncomfortable odor.

As a preparation of olmesartan medoxomil, a pharmaceutical package containing a tablet or capsule of olmesartan medoxomil in a bottle together with a desiccant, and a pharmaceutical package containing a blister pack housing a plurality of preparations of olmesartan medoxomil in an aluminum packaging bag together with a desiccant are used.
patent reference 1: WO2005/080384
patent reference 2: WO2006/107062
patent reference 3: JP-A-5-78328

### Disclosure of the Invention

### Problems to be Solved by the Invention

A preparation containing compound A or compound B can emit a specific odor because these compounds have a (5-methyl-2-oxo-1,3-dioxol-4-yl)methyl group (i.e., a medoxomil group) in a molecule. Since the odor of a preparation containing compound A or compound B is continuously generated as medoxomilester is gradually hydrolyzed, a pharmaceutical package capable of continuously removing the odor is demanded.

### Means of Solving the Problems

The present inventors have found that the odor of a preparation containing compound A or compound B can be decreased unexpectedly using a desiccant, which resulted in the completion of the present invention.
Accordingly, the present invention relates to
(1) a pharmaceutical package comprising a pharmaceutical preparation comprising 2-ethoxy-1-{[2'-(5-oxo-4,5-dihydro-1,2,4-oxadiazol-3-yl)biphenyl-4-yl]methyl)-1H-benzimidazole-7-carboxylic acid (5-methyl-2-oxo-1,3-dioxol-4-yl)methyl or 2-cyclopropyl-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl or a salt thereof, and a desiccant;
(2) the pharmaceutical package of the aforementioned (1), wherein the desiccant is synthetic zeolite, silica gel, silica alumina or activated carbon, or a mixture of two or more of these;
(3) a method of decreasing an odor of a pharmaceutical preparation comprising 2-ethoxy-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl or 2-cyclopropyl-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl or a salt thereof, which comprises using a desiccant;
(4) the method of the aforementioned (3), which comprises preserving a pharmaceutical preparation and a desiccant in a sealed package.

### Detailed Description of the Invention

Since a tautomer is present in the 5-oxo-4,5-dihydro-1,2,4-oxadiazol-3-yl group of compound A and compound B to be used in the present invention, compound A is also indicated as 2-ethoxy-1-{[2'-{5-oxo-2,5-dihydxo-1,2,4-oxadiazol-3-yl)biphenyl-4-yl]methyl}-1H-benzimidazole-7-carboxylic acid (5-methyl-2-oxo-1,3-dioxol-4-yl)methyl or a salt thereof. In addition, compound B is also indicated as 2-cyclopropyl-1 {[2'-(5-oxo-2,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl or a salt thereof (hereinafter compound A and compound B are sometimes collectively referred to as "the compound to be used in the present invention").

The compound to be used in the present invention can be produced according to the method disclosed in WO2005/080384 or WO2006/107062.

The compound to be used in the present invention also includes a pharmacologically acceptable salt thereof. Examples of such salt include salts with inorganic bases (e.g., alkali metals such as sodium, potassium etc., alkaline earth metals such as calcium, magnesium etc., transition metals such as zinc, iron, copper etc., and the like), and organic bases (e.g., organic amines such as trimethylamine, triethylamine, pyridine, picoline, tromethamine, ethanolamine, diethanolamine, triethanolamine, dicyclohexylamine, t-butylamine, N,N'-dibenzylethylenediamine and the like, basic amino acids such as arginine, lysine, ornithine etc.).

A pharmaceutical preparation containing compound A or compound B (hereinafter a pharmaceutical preparation containing compound A or compound B is sometimes referred to as "the preparation to be used in the present invention") may be any preparation containing compound A or compound B.

Examples of the dosage form of the preparation to be used in the present invention include solid dosage suitable for oral administration such as tablet, capsule, powder, granule, or fine granule.

The solid preparation can be produced according to a method known per se (e.g., the method described in the Japanese Pharmacopoeia 14th Revision, Preparation General Principles). For example, in the case of tablet, an active ingredient and an excipient (e.g., lactose, sucrose, glucose, starch, cornstarch, saccharose, microcrystalline cellulose, Glycyrrhiza uralensis, mannitol, sorbitol, sodium hydrogen carbonate, calcium phosphate, calcium sulfate etc.), and a disintegrant (e.g., amino acid, starch, cornstarch, calcium carbonate, carmellose sodium, carmellose calcium, croscarmellose sodium, low-substituted hydroxypropylcellulose, crospovidone, sodium carboxymethyl starch etc.) are mixed, a binder (e.g., hydroxypropylcellulose, hydroxypropylmethylcellulose, polyvinylpyrrolidone, gelatin, starch, gum arabic, tragacanth, carboxymethylcellulose, sodium alginate, pullulan, glycerol etc.) is added to give granules, and then, a lubricant (e.g., magnesium stearate, stearic acid, calcium stearate, purification talc etc.) and the like are added thereto and the mixture is tabletted to give tablets. In addition, granules and fine granules are obtained by granulation by an almost the same method as for tablet, or spraying water or a binder solution such as sucrose, hydroxypropylcellulose, hydroxypropylmethylcellulose and the like (concentration: about 0.5 - 70%(W/V)) on Nonpareil (trade name, spherical granules containing sucrose 75%(W/W) and cornstarch 25%(W/W)), while coating same with a powdery dusting agent comprising an active ingredient and an additive (e.g., sucrose, cornstarch, crystalline cellulose, hydroxypropylcellulose, methylcellulose, polyvinylpyrrolidone etc.). In the case of capsule, the above-mentioned granules and fine granules need only be filled in a capsule made of, for example, gelatin, hydroxypropylmethylcellulose and the like, or an active ingredient need only be filled in a capsule made of, for example, gelatin, hydroxypropylmethylcellulose and the like together with an excipient (e.g., lactose, sucrose, glucose, starch, saccharose, microcrystalline cellulose, Glycyrrhiza uralensis, mannitol, sodium hydrogen carbonate, calcium phosphate, calcium sulfate etc.).

The solid preparation may be coated with a coating agent for masking of taste, enteric property or sustained-release. Examples of the coating agent include hydroxypropylmethylcellulose, ethylcellulose, hydroxymethylcellulose, hydroxypropylcellulose, polyoxyethyleneglycol, Tween 80, pluronicF68, cellulose acetate phthalate, hydroxypropylmethylcellulose phthalate, hydroxymethylcellulose acetate succinate, Eudragit (manufactured by Rohm, Germany, methacrylic acid·acrylic acid copolymer) and where necessary, a light shielding agent such as titanium oxide or red iron oxide, can be used.

Examples of the "desiccant" to be used in the present invention include activated carbon, calcium chloride, silicon dioxide (silica gel), a bonded product of alumina oxide and silicon dioxide (silica alumina), alumina oxide (active alumina), natural or synthetic zeolite (molecular sieves 3A, 4A, 5A, 13X), allophane, clay, a mixture of silica gel and activated carbon, a mixture of silica gel and clay, a mixture of silica alumina and activated carbon, a mixture of synthetic zeolite and activated carbon, a mixture of allophane and activated carbon (e.g., allophane added with activated carbon, or allophane kneaded with activated carbon etc.), pulp containing silica gel (e.g., ultrafine silica gel mixed between paper fibers, silica gel packaged in paper tube etc.), pulp containing calcium chloride (e.g., paper material impregnated with liquid calcium chloride, dried and coated with film etc.) or pulp containing allophane (e.g., pulp impregnated with allophane liquid, dried and film coated, allophane packaged in paper tube etc.).

The "activated carbon" is a porous carbon substance having high specific surface area and adsorption capacity, which is produced from charcoal, coconut carbon, coal and the like, and used as adsorbent, catalyst carrier and the like. Preferably, the specific surface area is 800 - 1200 m²/g, fine pore volume is 0.2 - 2 cm³/g, and fine pore size is 1 - 4 nm. The composition is mainly carbon, and may further contain a small amount of hydrogen, oxygen and inorganic component. The chemical structure is basically graphite (black lead), or may be amorphous, and contain a functional group such as a hydroxyl group or a quinone group on the surface thereof.

Only one kind of the above-mentioned desiccant may be used or two or more kinds thereof may be used in combination.

In the present invention, synthetic zeolite, silica gel, silica alumina or activated carbon or a mixture of two or more of these are preferably used as the desiccant.

Of these, synthetic zeolite having high drying ability even under low humidity conditions is particularly preferable. In addition, when a package form such as a plastic bottle (polyethylene bottle etc.) is adopted, plastic bottles are moisture permeable and moisture inside plastic bottles may exceed 40%RH during the preservation period. Under such humidity conditions over 40%RH, silica gel has higher drying ability than synthetic zeolite. Thus, silica gel is particularly preferable.

The pharmaceutical package of the present invention characteristically reduces an odor caused by decomposition of compound A or compound B effectively since it contains a pharmaceutical preparation containing compound A or compound B and a desiccant. More particularly, the pharmaceutical preparation containing compound A or compound B and a desiccant preferably coexist independently in the pharmaceutical package of the present invention. As used herein, "coexist independently" means that a pharmaceutical preparation and a desiccant exist in the same space under a physically independent condition. As long as such conditions are satisfied, they may be in contact with each other or exist separately. In addition, as used herein, the "same space" means the inside space of a bottle or blister pack, and its size is not limited as long it can afford an odor decreasing effect. In addition, when a substance responsible for the odor can permeate a packaging material the pharmaceutical preparation and a desiccant are considered to be co-present in the same space even when they are separated by the packaging material.

In the pharmaceutical package of the present invention, the shape of a desiccant and the configuration of co-presence of the pharmaceutical preparation and a desiccant can be appropriately selected according to the dosage form and the configuration of packaging of the pharmaceutical preparation.

For example, when the pharmaceutical preparation is powder, granule, fine granule and the like, the desiccant is formed into pellet, plate, rod, tablet and the like having a sufficient size so that it is not mixed with the in pharmaceutical preparation and enclosed in a packaging container (e.g., glass bottle, plastic bottle (polyethylene bottle etc.), plastic bag (including one vapor-deposited with aluminum, silicon dioxide (silica) etc.), aluminum bag, metal can and a composite material thereof etc.), or the desiccant is formed into powder, granule, pellet, plate, rod, tablet and the like, packaged with a suitable gas permeable packaging material, such as known packaging materials (e.g., porous film made of a plastic sheet having fine pores, non-woven fabric, Japanese paper, foreign paper, glassine paper etc.) conventionally used for a packaging deoxidant or a carbon dioxide absorbent in packaging design of a pharmaceutical product, food and the like, or a canister and enclosed in a package container. When the package container has a form of a bottle with a cap, a desiccant packaged with the above-mentioned packaging material is preferably, but nonlimitatively, adhered to the backside of the cap.

When the pharmaceutical preparation is tablet or capsule, the desiccant can also be directly enclosed in the form of powder or granule in a package container, in addition to the embodiment usable for the above-mentioned powder, granule or fine granule. In addition, for packaging of tablet or capsule, a blister pack wherein a pharmaceutical preparation is placed in the cavity of a pan sheet generally made of a plastic or metal (e.g., aluminum etc.), and sealed with a cover sheet generally made of plastic or metal (e.g., aluminum etc.), is frequently used, where a pan sheet having further cavities for containing a desiccant in addition to the cavities for containing a pharmaceutical preparation (both cavities are not completely compartmented but have a communicating part permitting permeation of a causative substance of odor) may be formed, and a desiccant formed into a powder, granule or fine granule, pellet, plate, rod or tablet may be placed in the cavities for placing a desiccant and sealed with a plastic or aluminum material.

In the present invention, the "sealed package" is not particularly limited as long as it can house the preparation to be used in the present invention and a desiccant in a closed space, and includes the aforementioned package container (e.g., glass bottle, plastic bottle (polyethylene bottle etc.), a plastic bag (including one vapor-deposited with aluminum, silicon dioxide (silica) etc.), an aluminum bag, a metal can and a composite material thereof etc.) or a blister pack.

The amount of the desiccant to be used in the present invention is not particularly limited as long as it is sufficient to remove an odor substance (e.g., 2,3-butanedione (also called diacetyl) derived from compound A or compound B, that is, an amount sufficient to suppress or decrease an odor. In addition, the amount varies depending on the kind and form of the desiccant to be used, the distance from the pharmaceutical preparation, the amount and dosage form of compound A or compound B, the volume of the space in which the pharmaceutical preparation and the desiccant are placed, the amount of the odor substance present or to be produced or the preservation conditions of the pharmaceutical preparation. For example, when the desiccant of the present invention is used in a 200 ml container, the desiccant can be contained in an amount of about 50 mg - about 100 g, preferably about 300 mg - about 50 g, more preferably about 500 mg - about 20 g.

### Examples

In the following, the present invention is explained in detail by referring to Examples and Experimental Examples. However, they are mere examples and do not at all limit the scope of the present invention.

In the following Examples and Experimental Examples, 2-ethoxy-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl potassium salt (hereinafter to be referred to as compound a) and 2-cyclopropyl-1-{[2'-(5-oxo-4,5-dihydro-1,2,4-oxadiazol-3-yl)biphenyl-4-yl]methyll-1H-benzimidazole-7-carboxylic acid (5-methyl-2-oxo-1,3-dioxol-4-yl)methyl (hereinafter to be referred to as compound b) were used.

### EXAMPLES

### Formulation Example 1-1

### Preparation A-1

Compound a, granulated lactose (trade name Tablettose 80, MEGGLE JAPAN CO., LTD.), light anhydrous silicic acid (trade name AEROSIL) and magnesium stearate were mixed as powders in the following amounts.

**[Table 1]**

| | |
|---|---|
| Compound a | 53.35 g |
| granulated lactose | 343.85 g |
| light anhydrous silicic acid | 0.8 g |
| magnesium stearate | 2 g |

The powder mixture was filled in a No. 4 HPMC (hydroxypropylmethylcellulose) capsule by about 118.4 mg, and the obtained preparation (hereinafter to be referred to as preparation A-1) and a desiccant shown in the following Table 3 were placed in containers (glass vial or polyethylene bottle). The containers were tightly sealed to give preparations 1 - 5.

### Preparation A-2

Compound a, granulated lactose (trade name Tablettose 80, MEGGLE JAPAN CO., LTD.), light anhydrous silicic acid (trade name AEROSIL) and magnesium stearate were mixed as powders in the following amounts.

**[Table 2]**

| | |
|---|---|
| Compound a | 640.2 g |
| granulated lactose | 4102 g |
| light anhydrous silicic acid | 9.600 g |
| magnesium stearate | 48.00 g |

The powder mixture was filled in a No. 3 HPMC (hydroxypropylmethylcellulose) capsule by about 161.6 mg, and the obtained preparation (hereinafter to be referred to as preparation A-2) and a desiccant shown in the following Table 3 were placed in containers (glass vial or polyethylene bottle). The containers were tightly sealed to give preparations 6 - 8.

**[Table 3]**

| desiccant used | | | |
|---|---|---|---|
| | component of desiccant | amount of desiccant | form of desiccant/package thereof |
| (preparation 1) | molecular sieves 13X | 5.5 g | formed products 25×10/special Japanese paper package |
| (preparation 2) | silica alumina/activated carbon | 2 g | formed products 18×7/special Japanese paper package |
| (preparation 3) | silica alumina/activated carbon | 1 g | formed products 13×6/special Japanese paper package |
| (preparation 4) | silica alumina/activated carbon | 1 g | formed products 12×6/special Japanese paper package |
| (preparation 5) | silica gel/activated carbon | 3 g | particle/HDPE. container 25.2h×19.4φ |
| (preparation 6) | silica gel | 3 g | particle/HDPE container 25.2h×19.4φ |
| (preparation 7) | molecular sieves 4A | 3 g | Particle/HDPE container 25.2h×19.4φ |
| (preparation 8) | activated carbon | 2 g | powder/non-woven fabric bag 27×54 |

| | | | |
|---|---|---|---|
| The numbers are in mm. HDPE container: high density polyethylene container silica alumina/activated carbon: a mixture of silica alumina and activated carbon silica gel/activated carbon: a mixture of silica gel and activated carbon | | | |

### Formulation Example 1-2

### Preparation A-3

Compound a, granulated lactose (trade name FloLac 100, MEGGLE JAPAN CO., LTD.), light anhydrous silicic acid (trade name AEROSIL) and magnesium stearate were mixed as powders in the following amounts.

**[Table 4]**

| | |
|---|---|
| Compound a | 10.67 g |
| granulated lactose | 68.85 g |
| light anhydrous silicic acid | 0.16 g |
| magnesium stearate | 0.32 g |

The powder mixture was filled in a No. 3 HPMC capsule by about 146.8 mg, and the obtained preparation (hereinafter to be referred to as preparation A-3) and a desiccant shown in the following Table 5 were placed in containers (glass bottle). The containers were tightly sealed to give preparations 1' - 5'.

**[Table 5]**

| desiccant used | | | |
|---|---|---|---|
| | component of desiccant | amount of desiccant | form of desiccant/package thereof |
| (preparation 1') | molecular sieves 13X | 5.5 g | formed products 25×10/special Japanese paper package |
| (preparation 2') | silica alumina/activated carbon | 2 g | formed products 18×7/special Japanese paper package |
| (preparation 3') | silica alumina/activated carbon | 1 g | formed products 13×6/special Japanese paper package |
| (preparation 4') | silica alumina/activated carbon | 1 g | formed products 12×6/special Japanese paper package |
| (preparation 5') | silica gel/activated carbon | 3 g | particle/HDPE container 25.2h×19.4φ |

| | | | |
|---|---|---|---|
| The numbers are in mm HDPE container: high density polyethylene container Silica alumina/activated carbon: a mixture of silica alumina and activated carbon silica gel/activated carbon: a mixture of silica gel and activated carbon | | | |

### Formulation Example 2

**[Table 6]**

| composition | amount added (mg) |
|---|---|
| Compound b | 20 |
| mannitol | 78.8 |
| crystalline cellulose | 19.5 |
| hydroxypropylcellulose | 3.9 |
| croscarmellose sodium | 6.5 |
| magnesium stearate | 1.3 |
| plain tablet | 130 |
| hydroxypropylmethylcellulose | 3.735 |
| 2910 | |
| polyethylene glycol 6000 | 0.75 |
| titanium oxide | 0.5 |
| yellow diiron trioxide | 0.015 |
| total | 135 |

Compound b (689.7 g), mannitol (2670 g) and crystalline cellulose (663 g) were uniformly mixed in a fluidized bed granulator, granulated while spraying an aqueous solution of hydroxypropylcellulose (132.6 g) in the granulator and then dried therein. The obtained granules were pulverized using a power mill and a 1.5 mmφ punching screen to give milled granules. The milled granules were measured (3788 g), croscarmellose sodium (Ac-Di-Sol, 201.5 g) and magnesium stearate (40.3 g) were added and mixed to give granules for tabletting. The granules were tableted in a tabletting machine with a 7.0 mmφ punch to a weight of 130 mg to give plain tablets. A hydroxypropylmethylcellulose 2910 solution (solvent: purified water) obtained by dispersing titanium oxide and yellow ferric oxide and dissolving polyethylene glycol 6000 therein was sprayed on the obtained plain tablets in a film coating machine to give about 25000 film-coated tablets having the theoretical formulation shown in Table 6 and containing 20 mg of compound b per tablet.

The obtained preparation (hereinafter to be referred to as preparation B) and a desiccant shown in the following Table 7 were placed in containers (glass bottle). The containers were tightly sealed to give preparations 9 - 16.

**[Table 7]**

| desiccant used | | | |
|---|---|---|---|
| | component of desiccant | amount of desiccant | form of desiccant/package thereof |
| (preparation 9) | molecular sieves 13X | 5.5 g | formed products 25×10/special Japanese paper package |
| (preparation 10) | Silica alumina/activated carbon | 2 g | formed products 18×7/special Japanese paper package |
| (preparation 11) | Silica alumina/activated carbon | 1 g | formed products 13×6/special Japanese paper package |
| (preparation 12) | Silica alumina/activated carbon | 1 g | formed products 12×6/special Japanese paper package |
| (preparation 13) | silica gel /activated carbon | 3 g | particle/HDPE container 25.2h×19.4φ |
| (preparation 14) | silica gel | 3 g | particle/HDPE container 25.2h×19.4φ |
| (preparation 15) | molecular sieves 4A | 3 g | particle/HDPE container 25.2h×19.4φ |
| (preparation 16) | activated carbon | 2 g | powder/non-woven fabric bag 27×54 |

| | | | |
|---|---|---|---|
| The numbers are in mm. HDPE container: high density polyethylene container Silica alumina/activated carbon: a mixture of silica alumina and activated carbon silica gel/activated carbon: a mixture of silica gel and activated carbon | | | |

### Formulation Example 3 (placebo preparation)

**[Table 8]**

| composition | amount added (mg) |
|---|---|
| Compound b | 0 |
| mannitol | 98.8 |
| crystalline cellulose | 19.5 |
| hydroxypropylcellulose | 3.9 |
| croscarmellose sodium | 6.5 |
| magnesium stearate | 1.3 |
| plain tablet | 130 |
| hydroxypropylmethylcellulose | 3.735 |
| 2910 | |
| polyethylene glycol 6000 | 0.75 |
| titanium oxide | 0.5 |
| yellow diiron trioxide | 0.015 |
| total | 135 |

Mannitol (3359 g) and crystalline cellulose (663 g) were uniformly mixed in a fluidized bed granulator, granulated while spraying an aqueous solution of hydroxypropylcellulose (132.6 g) in the granulator and then dried therein. The obtained granules were pulverized using a power mill and a 1.5 mmφ punching screen to give milled granules. The milled granules were measured (3788 g), croscarmellose sodium (Ac-Di-Sol, 201.5 g) and magnesium stearate (40.3 g) were added and mixed to give granules for tabletting. The granules were tableted in a tabletting machine with a 7.0 mmφ punch to a weight of 130 mg to give plain tablets. A hydroxypropylmethylcellulose 2910 solution (solvent: purified water) obtained by dispersing titanium oxide and yellow ferric oxide and dissolving polyethylene glycol 6000 therein was sprayed on the obtained plain tablets in a film coating machine to give about 25000 film-coated tablets having the theoretical formulation shown in Table 8.

### Experimental Example 1

The preparations 1 - 8 prepared in the Examples were stored at 25°C 60%RH for 1, 2, 6 and 12 months, or at 40°C 75%RH for 1, 2, 3, 4 and 6 months, and the concentration of diacetyl in the containers, which is one of the odor components, was quantified by gas chromatography.
For sample Nos. 1-1 V and 1-1 P, preparation A-1 alone was placed in the containers.
For sample Nos. 1-2 V and 1-2 P, a placebo preparation (capsule filled with an excipient in the same amount as preparation A-1) alone was placed in the containers.
Each container was filled with 50 capsules.
glass vial: about 134 mL volume
polyethylene bottle: about 69 mL volume

Measurement conditions of gas chromatography
apparatus: Shimadzu GC-2010 gas chromatograph (Shimadzu Corporation)
detector: hydrogen flame ionization detector
analysis column: SPB-5 (manufactured by Supelco, 0.53 mm i.d.×30 m, membrane thickness: 5.0 µm)
column temperature: 80°C
carrier gas: helium
flow: 4.5 mL/min
inlet temperature: 200°C
detector temperature: 260°C
injection volume: 0.2 mL

As a result, the concentration (µg/mL) of diacetyl in the containers was as shown in the following Tables 9 and 10.

[Table 9]

**Table 9. 25°C 60%RH preservation test**

| sample No. | preparation | amount of diacetyl in headspace (µg/mL) | | | | |
|---|---|---|---|---|---|---|
| | | initial | 1 month | 2 months | 6 months | 12 months |
| 0V | control (empty bottle) | ND | ND | ND | ND | ND |
| OP | control (empty bottle) | ND | ND | ND | ND | ND |
| 1-1V | control (preparation A-1) | 0.01555 | 0.03267 | 0.03646 | 0.03630 | 0.02603 |
| 1--1P | control (preparation A-1) | 0.01915 | 0.03038 | 0.03913 | 0.06940 | 0.10529 |
| 1-2V | control (Placebo) | ND | ND | ND | ND | ND |
| 1-2P | control (Placebo) | ND | ND | ND | ND | ND |
| 1-3V | preparation 1 | ND | ND | 0.00220 | ND | ND |
| 1-3P | preparation 1 | ND | ND | ND | ND | ND |
| 2-3V | preparation 2 | ND | 0.00235 | ND | ND | ND |
| 2-3P | preparation 2 | ND | ND | ND | ND | ND |
| 3-3V | preparation 3 | ND | ND | ND | ND | ND |
| 3-3P | preparation 3 | ND | ND | ND | ND | ND |
| 4-3V | preparation 4 | ND | 0.00195 | ND | ND | ND |
| 4-3P | preparation 4 | ND | ND | ND | ND | ND |
| 5-3V | preparation 5 | 0.00111 | 0.00083 | ND | ND | ND |
| 5-3P | preparation 5 | 0.00017 | ND | ND | ND | ND |
| 6-3V | preparation 6 | ND | ND | ND | ND | ND |
| 6-3P | preparation 6 | ND | ND | ND | ND | ND |
| 7-3V | preparation 7 | ND | ND | ND | ND | ND |
| 7-3P | preparation 7 | 0.00318 | ND | ND | ND | ND |
| 8-3V | preparation 8 | ND | ND | ND | ND | ND |
| 8-3P | preparation 8 | ND | ND | ND | ND | ND |

| | | | | | | |
|---|---|---|---|---|---|---|
| V: glass vial, P: polyethylene bottle ND: not detected | | | | | | |

[Table 10]

**Table 10. 40°C 75%RH preservation test**

| sample No. | preparation | amount of diacetyl in headspace (µg/mL) | | | | | |
|---|---|---|---|---|---|---|---|
| | | initial | 1 month | 2 months | 3 months | 4 months | 6 months |
| 0V | control (empty bottle) | ND | ND | ND | ND | ND | ND |
| 0P | control (empty bottle) | ND | ND | ND | ND | ND | ND |
| 1-1V | control (preparation A-1) | 0.02806 | 0.07808 | 0.10281 | 0.08774 | 0.09411 | 0.09510 |
| 1-1P | control (preparation A-1) | 0.02293 | 0.16860 | 0.83033 | 0.88920 | 0.89139 | 0.66756 |
| 1-2V | control (Placebo) | ND | ND | ND | ND | ND | ND |
| 1-2P | control (Placebo) | ND | ND | ND | ND | ND | ND |
| 1-3V | preparation 1 | ND | ND | ND | ND | ND | ND |
| 1-3P | preparation 1 | ND | 0.03212 | ND | ND | ND | ND |
| 2-3V | preparation 2 | ND | ND | ND | ND | ND | ND |
| 2-3P | preparation 2 | ND | 0.01586 | ND | ND | ND | ND |
| 3-3V | preparation 3 | ND | 0.02078 | ND | ND | ND | ND |
| 3-3P | preparation 3 | ND | 0.00475 | ND | 0.00128 | 0.00143 | 0.01008 |
| 4-3V | preparation 4 | ND | 0.00266 | ND | ND | ND | ND |
| 4-3P | preparation 4 | ND | 0.00106 | ND | 0.00195 | 0.00176 | 0.02197 |
| 5-3V | preparation 5 | ND | 0.00029 | ND | ND | ND | ND |
| 5-3P | preparation 5 | ND | 0.00095 | ND | ND | ND | ND |
| 6-3V | preparation 6 | ND | ND | ND | ND | ND | ND |
| 6-3P | preparation 6 | ND | ND | ND | ND | ND | ND |
| 7-3V | preparation 7 | ND | ND | ND | ND | ND | ND |
| 7-3P | preparation 7 | ND | ND | ND | ND | ND | ND |
| 8-3V | preparation 8 | ND | ND | ND | ND | ND | ND |
| 8-3P | preparation 8 | ND | ND | ND | ND | ND | ND |

| | | | | | | | |
|---|---|---|---|---|---|---|---|
| V: glass vial, P: polyethylene bottle ND: not detected | | | | | | | |

In any preparation, the concentration of diacetyl, which is one of the odor components, remarkably decreased under the conditions generally used for the stability test of pharmaceutical products, and a deodorizing effect was obtained.

### Experimental Example 2 (sensory evaluation)

Preparation A-3 described in Formulation Example 1-2 was used as control preparation 1, and preparation A-2 described in Formulation Example 1-1 was used as control preparation 1'.
In addition, granulated lactose (trade name FloLac 100, MEGGLE JAPAN CO., LTD.) was filled in a No. 4 HPMC capsule by about 118 mg and used as placebo preparation 1.
Preparations 1' - 5', preparations 6 - 8, control preparation 1, control preparation 1' and placebo preparation 1 were placed in glass bottles (volume about 108 mL) by 4 capsules per 1 g of a desiccant.
Preparations 1' - 5', preparations 6 - 8, control preparation 1, control preparation 1' and placebo preparation 1 were stored at 25°C for 2 weeks, and the bottles were opened. A sensory evaluation of the odor upon opening was performed according the following evaluation criteria (n=3 or 4). Preparations 1' - 5' were compared with control preparation 1, and preparations 6 - 8 were compared with control preparation 1'.
1 point: No difference in odor from control preparation 1 or control preparation 1'
2 points: Slight deodorization as compared to control preparation 1 or control preparation 1'
3 points: Marked deodorization as compared to control preparation 1 or control preparation 1', but the odor is felt
4 points: Same as with placebo preparation 1, or completely deodorized.
As a result, average evaluation points of preparations 1' - 5' and preparations 6 - 8 were

| | |
|---|---|
| preparation 1' | 4 points |
| preparation 2' | 4 points |
| preparation 3' | 4 points |
| preparation 4' | 4 points |
| preparation 5' | 4 points |
| preparation 6 | 4 points |
| preparation 7 | 4 points |
| preparation 8 | 4 points. |

A deodorizing effect was obtained in all preparations.

### Experimental Example 3 (sensory evaluation)

Preparation B described in Formulation Example 2 was used as control preparation 2.
The preparation obtained in Formulation Example 3 was used as placebo preparation 2.
Preparations 9 - 16, control preparation 2 and placebo preparation 2 were placed in glass bottles (volume about 108 mL) by 4 capsules per 1 g of a desiccant.
Preparations 9 - 16, control preparation 2 and placebo preparation 2 were stored at 25°C for 2 weeks, and the glass bottles were opened. A sensory evaluation of the odor upon opening was performed according the following evaluation criteria (n=3).
1 point: No difference in odor from control preparation 2
2 points: Slight deodorization as compared to control preparation 2
3 points: Marked deodorization as compared to control preparation 2, but the odor is felt
4 points: Same as with placebo preparation 2, or completely deodorized.
As a result, average evaluation points of preparations 9 - 16 were

| | |
|---|---|
| preparation 9 | 4 points |
| preparation 10 | 4 points |
| preparation 11 | 4 points |
| preparation 12 | 4 points |
| preparation 13 | 4 points |
| preparation 14 | 4 points |
| preparation 15 | 4 points |
| preparation 16 | 4 points. |

A deodorizing effect was obtained in all preparations.

### Industrial Applicability

According to the present invention, the odor of a pharmaceutical preparation useful as a therapeutic drug for hypertension can be decreased, and the product value as a pharmaceutical product can be further enhanced.

## Claims

1. A pharmaceutical package comprising a pharmaceutical preparation comprising 2-ethoxy-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl or 2-cyclopropyl-1-{[2'-(5-oxo-4,5-dihydro-1,2,4-oxadiazol-3-yl)biphenyl-4-yl)methyl}-1H-benzimidazole-7-carboxylic acid (5-methyl-2-oxo-1,3-dioxol-4-yl)methyl or a salt thereof, and a desiccant.

2. The pharmaceutical package of claim 1, wherein the desiccant is synthetic zeolite, silica gel, silica alumina or activated carbon, or a mixture of two or more of these.

3. A method of decreasing an odor of a pharmaceutical preparation comprising 2-ethoxy-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl or 2-cyclopropyl-1-{[2'-(5-oxo-4,5-dihydro-1,2,4-oxadiazol-3-yl)biphenyl-4-yl]methyl}-1H-benzimidazole-7-carboxylic acid (5-methyl-2-oxo-1,3-dioxol-4-yl)methyl or a salt thereof, which comprises using a desiccant.

4. The method of claim 3, which comprises preserving a pharmaceutical preparation and a desiccant in a sealed package.

## Patentansprüche

1. Arzneimittelpackung mit einem pharmazeutischen Präparat, das 2-Ethoxy-1-{[2'-(5-oxo-4,5-dihydro-1,2,4-oxadiazol-3-yl)biphenyl-4-yl]methyl}-1H-benzimidazol-7-carbonsäure (5-methyl-2-oxo-1,3-dioxol-4-yl)methyl oder 2-Cyclopropyl-1-{[2'-(5-oxo-4,5-dihydro-1,2,4-oxadiazol-3-yl)biphenyl-4-yl]methyl}-1 H-benzimidazol-7-carbonsäure (5-methyl-2-oxo-1,3-dioxol-4-yl)methyl oder ein Salz davon sowie ein Trockenmittel umfasst.

2. Arzneimittelpackung nach Anspruch 1, wobei das Trockenmittel synthetischer Zeolit, Kieselgel, Silica-Aluminiumoxid oder Aktivkohle oder eine Mischung von zwei oder mehreren davon ist.

3. Verfahren zum Vermindern eines Geruchs eines pharmazeutischen Präparats, das 2-Ethoxy-1-{[2'-(5-oxo-4,5-dihydro-1,2,4-oxadiazol-3-yl)biphenyl-4-yl]methyl}-1H-benzimidazol-7-carbonsäure (5-methyl-2-oxo-1,3-dioxol-4-yl)methyl oder 2-Cyc!opropyl-1-{[2'-(5-oxo-4,5-dihydro-1,2,4-oxadiazol-3-yl)bipheny)-4-yl]methyl}-1H-benzimidazol-7-carbonsäure (5-methyl-2-oxo-1,3-dioxol-4-yl)methyl oder ein Salz davon umfasst, bei dem ein Trockenmittel verwendet wird.

4. Verfahren nach Anspruch 3, bei dem ein pharmazeutisches Präparat und ein Trockenmittel in einer versiegelten Packung konserviert werden.

## Revendications

1. Emballage pharmaceutique, contenant une préparation pharmaceutique comprenant du 2-éthoxy-1-{[2'-(5-oxo-4,5-dihydro-1,2,4-oxadiazol-3-yl)-biphényl-4-yl]-méthyl}-1H-benzimidazole-7-carboxy-late de (5-méthyl-2-oxo-1,3-dioxol-4-yl)-méthyle ou du 2-cyclopropyl-1-{[2'-(5-oxo-4,5-dihydro-1,2,4-oxadiazol-3-yl)-biphényl-4-yl]-mé-thyl}-1H-benzimidazole-7-carboxylate de (5-méthyl-2-oxo-1,3-dioxol-4-yl)-méthyle ou un sel de l'un de ces composés, ainsi qu'un agent desséchant.

2. Emballage pharmaceutique conforme à la revendication 1, dans lequel l'agent desséchant est une zéolithe synthétique, un gel de silice, une silice-alumine ou un charbon actif, ou un mélange de deux de ces corps ou plus.

3. Procédé permettant de réduire l'odeur d'une préparation pharmaceutique comprenant du 2-éthoxy-1-{[2'-(5-oxo-4,5-dihydro-1,2,4-oxadiazol-3-yl)-biphényl-4-yl]-méthyl}-1H-benzimidazole-7-carboxy-late de (5-méthyl-2-oxo-1,3-dioxol-4-yl)-méthyle ou du 2-cyclopropyl-1-{[2'-(5-oxo-4,5-dihydro-1,2,4-oxadiazol-3-yl)-biphényl-4-yl]-méthyl}-1H-benzimidazole-7-carboxylate de (5-méthyl-2-oxo-1,3-dioxol-4-yl)-méthyle ou un sel de l'un de ces composés, lequel procédé comporte l'utilisation d'un agent desséchant.

4. Procédé conforme à la revendication 3, qui comporte le fait de conserver la préparation pharmaceutique et l'agent desséchant dans un emballage hermétiquement fermé.
